(19) Europäisches Patentamt
European Patent Office
Office européen des brevets

(11) Veröffentlichungsnummer: **0 133 156 B1**

(12) # EUROPÄISCHE PATENTSCHRIFT

(45) Veröffentlichungstag der Patentschrift: **10.07.91**

(51) Int. Cl.$^5$: **C07D 487/04**, C09B 69/00, C09B 57/00, D06P 3/54, C08K 5/34, //(C07D487/04, 209:00,209:00)

(21) Anmeldenummer: **84810310.7**

(22) Anmeldetag: **25.06.84**

Die Akte enthält technische Angaben, die nach dem Eingang der Anmeldung eingereicht wurden und die nicht in dieser Patentschrift enthalten sind.

(54) **Neue 1,4-Diketopyrrolo-(3,4-c)-pyrrole.**

(30) Priorität: **29.06.83 CH 3568/83**

(43) Veröffentlichungstag der Anmeldung:
**13.02.85 Patentblatt 85/07**

(45) Bekanntmachung des Hinweises auf die Patenterteilung:
**10.07.91 Patentblatt 91/28**

(84) Benannte Vertragsstaaten:
**CH DE FR GB IT LI**

(56) Entgegenhaltungen:
**EP-A- 0 061 426**
**EP-A- 0 094 911**
**EP-A- 0 098 808**
**EP-A- 0 102 318**

**CHEMICAL ABSTRACTS, Band 82, Nr. 1, 6. Januar 1975, Seite 359, Zusammenfassungsnr. 4148v, Columbus, Ohio, US; D.G. FARNUM et al.: "Attempted reformatskii reaction of benzonitrile. 1,4-Dioxo-3,6-diphenylpyrrolo[3,4-c]pyrrole, a lactam analog of pentalene"**

**JOURNAL OF THE CHEMICAL SOCIETY, PERKIN TRANSACTIONS I, Nr. 1. 1976, Seiten 5-8; J.M. SPRAKE et al.: "Addition products of 2-(N-Arylformimi doyl)pyridines and carbanions, and their reduction to Octahydroindolizine derivatives"**

(73) Patentinhaber: **CIBA-GEIGY AG**
**Klybeckstrasse 141**
**CH-4002 Basel(CH)**

(72) Erfinder: **Jost, Max**
**Rebgartenweg 20**
**CH-4104 Oberwil(CH)**
Erfinder: **Iqbal, Abul, Dr.**
**Im Schaiengarten 1**
**CH-4107 Ettingen(CH)**
Erfinder: **Rochat, Alain Claude, Dr.**
**route de Schiffenen 38**
**CH-1700 Fribourg(CH)**

EP 0 133 156 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

EP-A-0061426 beschreibt Pyrrolopyrrole, die an den Stickstoffatomen der Pyrrolringe unsubstituiert sind und als Pigmente zum Färben von Polymeren vorgeschlagen werden. Derartige Verbindungen können z.B. nach den in EP-A-0094911, EP-A-0098808 und EP-A-0102318 beschriebenen Verfahren gewonnen werden.

Die vorliegende Erfindung betrifft neue 1,4-Diketopyrrolo-[3,4-c]-pyrrole der Formel I

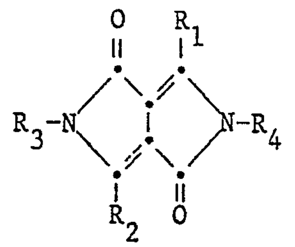

(I),

worin $R_1$ und $R_2$ einen Rest der Formel II

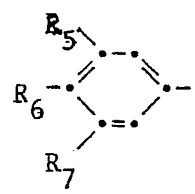

(II)

bedeuten, worin $R_5$, $R_6$ und $R_7$ unabhängig voneinander Wasserstoff, Halogen, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$-Monoalkylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_5$, $R_6$ und $R_7$ Wasserstoff bedeutet, und $R_3$ und $R_4$ unabhängig voneinander $C_1$-$C_{12}$-Alkyl, Cyanmethyl, $C_2$-$C_{13}$-Alkoxycarbonyl, Carbamoyl, $C_2$-$C_{13}$-Alkylcarbamoyl, $C_2$-$C_{13}$-Alkyl-$C_1$-$C_4$-alkoxycarbonyl,unsubstituiertes oder durch Halogen, $C_1$-$C_{12}$-Alkyl, $C_1$-$C_{12}$-Alkoxy, Trifluormethyl oder Nitro substituiertes Phenyl, Benzoyl oder Benzyl, oder die Gruppe der Formel

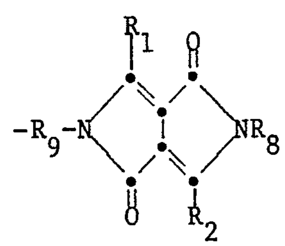

bedeuten, worin $R_1$ und $R_2$ die oben angegebene Bedeutung haben, $R_8$ Wasserstoff bedeutet und $R_9$ einen zweiwertigen Rest bedeutet, der sich von einem $C_1$-$C_8$-Dihaloalkan, $C_1$-$c_{10}$-Dicarbonsäurehalogenid, Phenylendihalogenid, oder Xylylendihalogenid ableitet,
wobei entweder $R_3$ oder $R_4$ auch Wasserstoff sein kann.

Formel (I) sowie die nachfolgend aufgeführten Formeln geben nur eine der möglichen tautomeren Strukturen wieder.

In den oben für $R_1$ und $R_2$ angegebenen Definitionen bedeuten:

a) Halogenatome, beispielsweise Chlor, Brom oder Fluor,

b) $C_1$-$C_{12}$-Alkyl verzweigte oder unverzweigte Alkylgruppen, wie z.B. Methyl, Aethyl, n-Propyl, Isopropyl, n-Butyl, sec-Butyl, tert.-Butyl, tert.-Amyl, n-Pentyl, n-Hexyl, 1,1,3,3-Tetramethylbutyl, n-Heptyl, n-Octyl, Nonyl, Decyl, Undecyl oder Dodecyl,

c) $C_1$-$C_{12}$-Alkoxy oder Phenoxy beispielsweise Methoxy, Aethoxy, n-Propoxy, Isopropoxy, Phenoxy, o-,

m- oder p-Chlorphenoxy, o-, m- oder p-Methylphenoxy,

d) $C_1$-$C_{12}$-Alkylmercapto oder Phenylmercapto z.B. Methyl-, Aethyl-, n-Propyl-, Isopropyl-, Phenyl-, o-, m- oder p-Chlorphenyl-, o-, m- oder p-Methylphenylmercapto,

e) $C_1$-$C_{12}$-Monoalkylamino und $C_2$-$C_{24}$-Dialkylamino z.B. Methylamino, Dimethylamino, Aethylamino, Diäthylamino oder Isopropylamino,

f) $C_2C_{13}$-Alkoxycarbonyl z.B. Methoxy-, Aethoxy-, Isopropoxy-, tert.-Butoxy-oder n-Butoxy-carbonyl,

g) $C_2$-$C_{13}$-Alkanoylamino oder Benzoylamino z.B. Acetylamino, Propionylamino, Butyrylamino, Benzoylamino, p-Chlorbenzoylamino oder p-Methylbenzoylamino,

h) Phenylcarbamoyl wie N-Phenylcarbamoyl.

$R_3$ und $R_4$ als Substituenten können insbesondere bedeuten:

1) Verzweigte oder unverzweigte $C_1$-$C_{12}$-Alkylgruppen wie oben unter b) angegeben, ferner Cyanmethyl;

2) $C_2$-$C_{13}$-Alkylcarbamoyl, wie N-Methylcarbamoyl, N-Aethylcarbamoyl;

3) Unsubstituiertes oder durch Halogen, wie Chlor oder Brom, Nitro, Trifluormethyl, $C_1$-$C_{12}$-Alkyl oder $C_1$-$C_{12}$-Alkoxy substituiertes Phenyl, wie Phenyl, 4-Methylphenyl, 2,4-Dichlorphenyl, 4-Chlorphenyl, 4-Nitrophenyl oder 3-Methoxyphenyl;

4) Die Gruppe der Formel

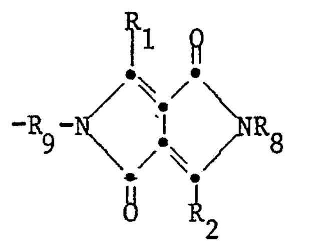 ,

worin $R_1$ und $R_2$ die oben angegebenen Bedeutungen haben, $R_8$ Wasserstoff bedeutet und $R_9$ einen zweiwertigen Rest bedeutet, der sich von einem $C_1$-$C_8$-Dihaloalkan, wie Cl $(CH_2)_2Cl$, $C_1$-$C_{10}$-Dicarbonsäurehalogenid, wie $ClCO(CH_2)_4COCl$, von einem Phenylendihalognid, oder einem Xylylendihalogenid ableitet. Halogen kann insbesondere Chlor oder Brom bedeuten.

Von besonderem Interesse sind Verbindungen der Formel I, worin $R_3$ und $R_4$ beide verschieden von Wasserstoff sind und den gleichen Substituenten bedeuten.

Besonders bevorzugte Verbindungen entsprechen der Formel I, worin $R_1$ und $R_2$ einen Rest der Formel III

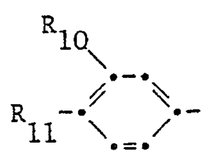 (III)

bedeuten, worin einer der Substituenten $R_{10}$ und $R_{11}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes Phenylcarbamoyl bedeutet, und der andere Wasserstoff ist.

Besonders bevorzugte Verbindungen entsprechen der Formel I, worin $R_3$ und $R_4$ $C_1$-$C_8$-Alkyl, $C_2$-$C_5$-Alkoxycarbonyl, Carbamoyl, $C_2$-$C_{13}$-Alkyl-$C_1$-$C_4$-alkoxycarbonyl, unsubstituiertes oder durch Chlor, Brom, $C_1$-$C_4$-Alkyl, $C_1$-$C_4$-Alkoxy, Trifluormethyl oder Nitro substituiertes Phenyl bedeuten.

Von besonderem Interesse sind Verbindungen der Formel I, worin $R_1$ und $R_2$ einen Rest der Formel

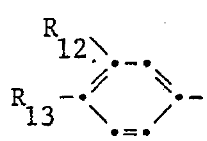 (IV)

bedeuten, worin einer der Substituenten $R_{12}$ und $R_{13}$ $C_1$-$C_4$-Alkyl und der andere Wasserstoff bedeutet,

und $R_3$ und $R_4$ $C_1$-$C_8$-Alkyl bedeuten.

Von weiterem Interesse sind Verbindungen der Formel I, worin $R_1$ und $R_2$ einen Rest der Formel

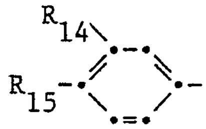

(V)

bedeuten, worin einer der Substituenten $R_{14}$ und $R_{15}$ Cyano und der andere Wasserstoff bedeuten, und $R_3$ und $R_4$ $C_1$-$C_4$-Alkyl bedeuten.

Die Verbindungen der Formel I können auf verschiedene Art hergestellt werden, beispielsweise

a) durch Umsetzen der Verbindung der Formel

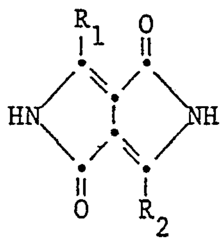

(VI)

mit einer die Reste $R_3$ bzw. $R_4$ als Abgangsgruppen enthaltenden Verbindung in einem organischen Lösungsmittel, oder

b) durch Umsetzen von 2 Mol einer Verbindung der Formel

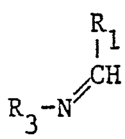

(VII) bzw.

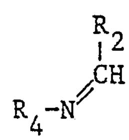

(VIII)

oder von je einem Mol der Verbindungen der Formel (VII) und (VIII) mit 1 Mol eines Bernsteinsäurediesters in Gegenwart von Base und einem organischen Lösungsmittel (vgl. J.Chem.Soc. 1976, S. 5) und anschliessendes Dehydrieren.

Die Ausgangsverbindungen der Formeln (VI), (VII) und (VIII) sind bekannt und können nach bekannten Verfahren hergestellt werden.

Je nach Art ihrer Substituenten und des zu färbenden Polymeren können die Verbindungen der Formel I insbesondere als polymerlösliche Farbstoffe für z.B. Polystyrol, Polyamide, ABS, vor allem für lineare Polyester, oder auch als Pigmente für hochmolekulares organisches Material verwendet werden. Als lineare Polyester seien insbesondere jene erwähnt, die durch Polykondensation von Terephthalsäure oder deren Estern mit Glykolen der Formel HO-$(CH_2)_n$-OH, worin n die Zahl 2-10 bedeutet, oder mit 1,4-Di-(hydroxymethyl)-cyclohexan oder durch Polykondensation von Glykoläthern von Hydroxybenzoesäuren, beispielsweise p-($\beta$-Hydroxyäthoxy)-benzoesäure erhalten werden. Der Begriff lineare Polyester umfasst auch Kopolyester, die durch teilweisen Ersatz der Terephthalsäure durch eine andere Dicarbonsäure und/oder durch teilweisen Ersatz des Glykols durch ein anderes Diol erhalten werden.

Von besonderem Interesse sind jedoch die Polyäthylenterephthalate.

Die zu färbenden linearen Polyester werden zweckmässig in Form von Pulver, Schnitzeln oder Granulaten mit dem Farbstoff innig vermischt. Dies kann beispielsweise durch Panieren der Polyesterteilchen mit dem fein verteilten trockenen Farbstoffpulver oder durch Behandeln der Polyesterteilchen mit einer Lösung bzw. Dispersion des Farbstoffes in einem organischen Lösungsmittel und nachheriger Entfernung des Lösungsmittels geschehen.

Die zu färbende Substanz kann auch nach der Badfärbung eingefärbt werden.

Zur Nuanceeinstellung können Gemische der Verbindungen der Formel I sowie Gemische einer oder mehrerer Verbindungen der Formel I mit Dispersionsfarbstoffen verwendet werden.

Schliesslich kann die Verbindung der Formel I auch direkt dem geschmolzenen Polyester oder auch vor oder während der Polykondensation des Polyäthylenterephthalates zugegeben werden.

Das Verhältnis von Farbstoff zu Polyester kann, je nach der gewünschten Farbstärke, innerhalb weiter Grenzen schwanken. Im allgemeinen empfiehlt sich die Verwendung von 0,01-3 Teilen Farbstoff auf 100 Teile Polyester.

Die so behandelten Polyesterteilchen werden nach bekannten Verfahren im Extruder geschmolzen und zu Gegenständen, insbesondere Folien oder Fasern, ausgepresst oder zu Platten gegossen.

Im Falle der Verwendung als Pigmente ist es vorteilhaft, die bei der Synthese anfallenden Produkte in eine feindisperse Form überzuführen. Dies kann auf verschiedene Weise geschehen, beispielsweise:

a) durch Mahlen oder Kneten, zweckmässig in Gegenwart von Mahlhilfsmitteln, wie anorganischen oder organischen Salzen mit oder ohne Zusatz organischer Lösungsmittel. Nach dem Mahlen werden die Hilfsmittel wie üblich entfernt, lösliche anorganische Salze z.B. mit Wasser und wasserunlösliche organische Lösungsmittel beispielsweise durch Wasserdampfdestillation,

b) durch Umfällen aus Schwefelsäure, Methansulfonsäure, Trichloressigsäure oder Polyphosphorsäure.

c) im Falle von Produkten, in denen $R_3$ oder $R_4$ Wasserstoff ist, durch Ueberführen des Rohpigmentes in ein Alkali- oder Aminsalz und Hydrolyse des letzteren. Dies geschieht beispielsweise durch Anrühren des Rohpigmentes mit einer Base, beispielsweise mit einem Alkalihydroxid, oder -alkoholat, Ammoniak oder einem Amin in einem polaren organischen Lösungsmittel wie Dimethylformamid, wobei das Pigment ganz oder teilweise in Lösung geht. Durch Hydrolyse, vorzugsweise durch Ansäuern der gegebenenfalls filtrierten Lösung wird das Pigment ausgefällt.

Es kann sich als zweckmässig erweisen, die nach a), b) oder c) behandelten Pigmente mit organischen Lösungsmitteln, vorzugsweise mit solchen, die über 100 °C sieden, nachzubehandeln.

Als besonders geeignet erweisen sich durch Halogenatome, Alkyl- oder Nitrogruppen substituierte Benzole, wie Xylole, Chlorbenzol-, o-Dichlorbenzol oder Nitrobenzol sowie Pyridinbasen, wie Pyridin, Picolin oder Chinolin, ferner Ketone wie Cyclohexanon, Aether wie Aethylenglykolmonomethyl-oder -monoäthyläther, Amide wie Dimethylformamid oder N-Methyl-pyrrolidon, sowie Dimethylsulfoxid, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen, oder aliphatischen Aminen oder mit flüssigem Ammoniak durchführen.

Je nach Verwendungszweck erweist es sich als vorteilhaft, die Pigmente als Toner oder in Form von Präparaten zu verwenden.

Das hochmolekulare organische Material kann natürlicher oder künstlicher Herkunft sein. Es kann sich z.B. um Naturharze oder trocknende Oele, Kautschuk oder Casein oder um abgewandelte Naturstoffe, wie Chlorkautschuk, ölmodifizierte Alkydharze, Viscose, um Celluloseäther oder Ester, wie Celluloseacetat, Cellulosepropionat, Celluloseacetobutyrat oder Nitrocellulose handeln, insbesondere aber um vollsynthetische organische Polymere (Duroplaste und Thermoplaste), wie sie durch Polymerisation, Polykondensation oder Polyaddition erhalten werden. Aus der Klasse der Polymerisationsharze seien in erster Linie genannt, Polyolefine, wie Polyäthylen, Polypropylen, oder Polyisobutylen, ferner substituierte Polyolefine, wie Polymerisate aus Vinylchlorid, Vinylacetat, Styrol, Acrylnitril, Acrylsäure- und/oder Methacrylsäureestern oder Butadien, sowie Kopolymerisate der erwähnten Monomeren, insbesondere ABS oder EVA.

Aus der Reihe der Polyadditions- und Polykondensationsharze seien die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste, die als Lackharze verwendeten Polyester, und zwar sowohl gesättigte, wie z.B. Alkydharze, als auch ungesättigte, wie beispielsweise Maleinatharze, ferner die linearen Polyester und Polyamide oder Silikone, genannt.

Die erwähnten hochmolekularen Verbindungen können einzeln oder in Gemischen, als plastische Hassen oder Schmelzen, die gegebenenfalls zu Fasern versponnen werden können, vorliegen.

Sie können auch in Form ihrer Monomeren oder im polymerisierten Zustand in gelöster Form als Filmbildner oder Bindemittel für Lacke oder Druckfarben vorliegen, wie z.B. Leinölfirnis, Nitrocellulose, Alkydharze, Melaminharze und Harnstoff-Formaldehydharze oder Acrylharze.

Die Pigmentierung der hochmolekularen, organischen Substanzen mit den Pigmenten der Formel (I) erfolgt beispielsweise derart, dass man ein solches Pigment gegebenenfalls in Form von Masterbatches diesen Substraten unter Verwendung von Walzwerken, Misch- oder Mahlapparaten zumischt. Das pigmentierte Material wird hierauf nach an sich bekannten Verfahren wie Kalandrieren, Pressen, Strangpressen, Streichen, Giessen oder Spritzguss in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemässen Verfahren vor oder nach der Einverleibung des Pigmentfarbstoffes in die Polymeren eingearbeitet werden. Es ist ferner möglich, zwecks Erzielung verschiedener Farbtöne den hochmolekularen, organischen

Stoffen neben den Verbindungen der Formel (I) noch Füllstoffe bzw. andere farbgebende Bestandteile wie Weiss-, Bunt- oder Schwarzpigmente in beliebigen Mengen zuzufügen.

Zum Pigmentieren von Lacken und Druckfarben werden die hochmolekularen organischen Materialien und die Verbindungen der Formel (I) gegebenenfalls zusammen mit Zusatzstoffen wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch einen gelben bis roten Farbton, eine sehr grosse Farbstärke, hohe Sättigung, gute Dispergierbarkeit, gute Ueberlackier-, Migrations-, Hitze-, Licht- und Wetterechtheit sowie durch einen guten Glanz und gutes IR-Remissionsverhalten aus.

Die Verbindungen der Formel (I) können auch als photoelektrophoretische Toner verwendet werden.

Wenn die Verbindungen der Formel (I) in den angewandten Polymeren gelöst vorliegen, zeichnen sie sich ebenfalls durch reinen Farbton, grosse Farbstärke, gute Echtheiten, insbesondere Licht- und Sublimierechtheit, und ausserdem durch hohe Fluoreszenz aus. Sie eignen sich in Sonnenenergie-Kollektoren und zur Herstellung von Laser-Strahlen.

In den folgenden Beispielen sind Teile Gewichtsteile und Prozente Gewichtsprozente, sofern nichts anderes angegeben ist.

Beispiel 1a: 14,5 Teile 1,4-Diketo-3,6-diphenylpyrrolo-[3,4-c]-pyrrol, 15 Teile wasserfreies Kaliumcarbonat, 40 Teile p-Toluolsulfonsäuremethylester und 170 Teile trockenes Nitrobenzol werden eine Stunde unter Rühren auf 200-205° C erhitzt. Nach Abkühlen auf 20° C wird das entstandene 1,4-Diketo-2,5-dimethyl-3,6-diphenylpyrrolo-[3,4-c]-pyrrol abfiltriert, mit Toluol, dann Methanol, und darauf mit heissem Wasser gewaschen und getrocknet. Man erhält 10,85 Teile orange Kristalle der Verbindung der Formel (IX) (Smp. 236-238° ).

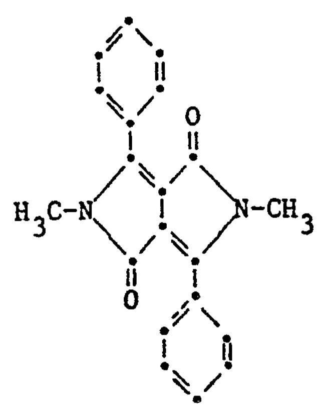

(IX) .

Beispiel 1b: 1 Teil des gemäss Beispiel 1a erhaltenen Farbstoffes wird mit 2 Teilen einer 50%igen wässrigen Lösung des Natriumsalzes der Dinapthylmethandisulfonsäure nass vermahlen und getrocknet.

0,1 Teile dieses Farbstoffpräparates werden mit 0,1 Teilen Oleylmethyltaurid-Natriumsalz, 0,1 Teilen Dinaphthylmethandisulfonsäure-Natriumsalz und 0,5 Teilen Ammoniumsulfat verrührt. Durch Verdünnen mit Wasser wird daraus ein Färbebad von 200 Teilen bereitet und dessen pH-Wert mit 85%iger wässriger Ameisensäure auf 5 gestellt. In dieses Bad geht man bei 50° C mit 10 Teilen eines Diolen-Gewebes (Polyester) ein, steigert die Temperatur innert 30 Minuten im geschlossenen Gefäss auf 120° C und dann innerhalb weiterer 10 Minuten auf 130° C. Anschliessend wird gut gespült. Man erhält eine kräftige gelbe Färbung, wobei der Farbstoff sehr gut aus dem Färbebad ausgezogen ist.

Beispiel 2: 6,8 Teile 1,4-Diketo-3,6-di-(3'-cyanophenyl)-pyrrolo-[3,4-c]-pyrrol, 6 Teile wasserfreies Kaliumcarbonat und 16 Teile p-Toluolsulfonsäuremethylester werden in 100 Teilen trockenem Nitrobenzol 2 Stunden unter Rühren auf 200-205° C erhitzt. Der entstandene Farbstoff wird nach dem Erkalten der Suspension abfiltriert, mit Nitrobenzol, Aceton und schliesslich mit heissem Wasser gewaschen. Nach dem Trocknen erhält man 5,5 Teile 1,4-Diketo-2,5-dimethyl-3,6-di-(3'-cyanophenyl)-pyrrolo-[3,4-c]-pyrrol [Formel (X)] in Form von braunroten Kristallen. Der Farbstoff färbt nach Ueberführen in feine Verteilung PVC und Lacke in roten Tönen von guter Licht- und Migrationsechtheit.

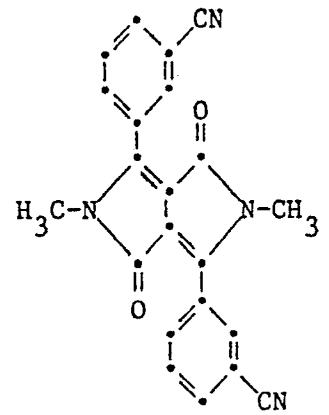

(X)

Beispiel 3: Verwendet man statt 6,8 Teilen 1,4-Diketo-3,6-di-(3'-cyanophenyl)-pyrrolo-[3,4-c]-pyrrol 6,8 Teile 1,4-Diketo-3,6-di-(4'-cyanophenyl)-pyrrolo-[3,4-c]-pyrrolund arbeitet sonst wie in Beispiel 2 beschrieben, so erhält man 5,5 Teile der Verbindung der Formel XI

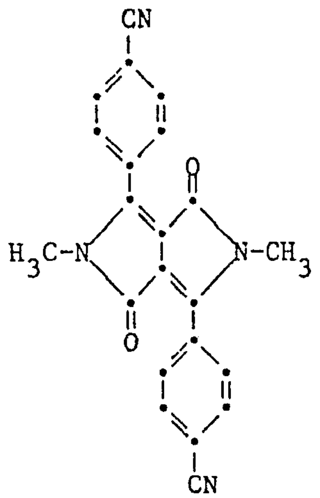

(XI) ,

die PVC und Lacke in braunorangen Tönen von guter Licht- und Migrationsechtheit färbt.

Beispiel 4:

4a) Zur Suspension von 7,2 Teilen 1,4-Diketo-3,6-di-(3'-chlorphenyl)-pyrrolo[3,4-c]-pyrrol und 11,1 Teilen Kaliumcarbonat in 70 Teilen Dimethylformamid wird bei 135° C unter Rühren eine Lösung von 16 Teilen Toluol-4-sulfonsäuremethylester in 30 Teilen Dimethylformamid in 40 Minuten zugetropft. Die Mischung wird 10 Minuten bei 135-140° C nachgerührt, alsdann auf 20° C abgekühlt und filtriert. Das abfiltrierte Produkt wird zweimal mit wenig Dimethylformamid, darauf mit Methanol und 90° C heissem Wasser gewaschen. Das Rohprodukt wird in Wasser suspendiert, die Suspension zum Sieden erhitzt und filtriert. Zur Abtrennung von nicht umgesetztem Ausgangsmaterial wird das getrocknete und pulverisierte Rohprodukt mit 175 Teilen Toluol zum Sieden erhitzt und die filtrierte Lösung nach Abkühlen auf 15° C mit 400 Teilen Petroläther (Kp. 40 - 60° C) versetzt. Das ausfallende 1,4-Diketo-2,5-dimethyl-3,6-di-(3'-chlorphenyl)-pyrrolo[3,4-c]-pyrrol (Formel XII) wird abfiltriert und getrocknet. Es stellt ein orangerotes Pulver mit dem Schmelzpunkt 226 - 227° C dar. Die gelbe Lösung in Dimethylformamid zeigt ausgeprägte gelbe Fluoreszens. Der Farbstoff färbt nach der im Beispiel 1b beschriebenen Methode PES in reinen gelben Tönen von guter Licht- und Sublimierechtheit.

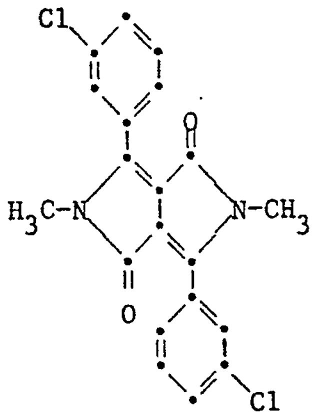

(XII)

4b) Ersetzt man Toluol-4-sulfonsäuremethylester durch Toluol-4-sulfonsäureäthylester, so erhält man 1,4-Diketo-2,5-diäthyl-3,6-di-(3'-chlorphenyl)-pyrrolo-[3,4-c]-pyrrol in Form eines gelben Pulvers vom Fp. 203 - 205 ° C mit ähnlichen Eigenschaften.

Beispiel 5: 9,5 Teile 1,4-Diketo-3,6-di-(3'-methylphenyl)-pyrrolo-[3,4-c]-pyrrol, 9 Teile wasserfreies Kaliumcarbonat und 24 Teile p-Toluolsulfonsäuremethylester werden mit 85 Teilen Nitrobenzol 1 Stunde unter Rühren auf 190 ° C erhitzt. Die abgekühlte Reaktionsmischung wird filtriert. Der gebildete Farbstoff bleibt im Lösungsmittel gelöst. Mittels Wasserdampfdestillation wird das Nitrobenzol entfernt und der Rohfarbstoff durch Kristallisation aus 45 Teilen Toluol gereinigt. Man erhält 3,8 Teile 1,4-Diketo-2,5-dimethyl-3,6-di(3'-methylphenyl)-pyrrolo-[3,4-c]-pyrrol in Form oranger Kristalle vom Smp. 195-197 ° C (Formel XIII). Der Farbstoff färbt PES nach der in Beispiel 1b angegebenen Methode in reinem gelben Ton von guten Echtheitseigenschaften.

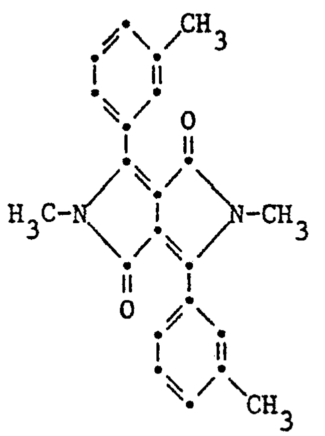

(XIII)

Beispiel 6: 9,5 Teile 1,4-Diketo-3,6-di-(4'-methylphenyl)-pyrrolo-[3,4-c]-pyrrol, 9 Teile wasserfreies Kaliumcarbonat, 24 Teile p-Toluolsulfonsäuremethylester und 110 Teile Nitrobenzol werden 1/2 Stunde bei 200 ° C gerührt. Nach Erkalten der Mischung wird der Farbstoff abfiltriert, mit wenig Nitrobenzol, alsdann mit Methanol und heissem Wasser gewaschen. Nach dem Trocknen erhält man 5,8 Teile 1,4-Diketo-2,5-dimethyl-3,6-di-(4'-methylphenyl)-pyrrolo-[3,4-c]-pyrrol in Form gelbstichig roter Kristalle vom Smp. 260 ° C (Formel XIV). Der Farbstoff färbt PES nach der in Beispiel 1 beschriebenen Methode in reinen gelben Tönen von guten Echtheitseigenschaften.

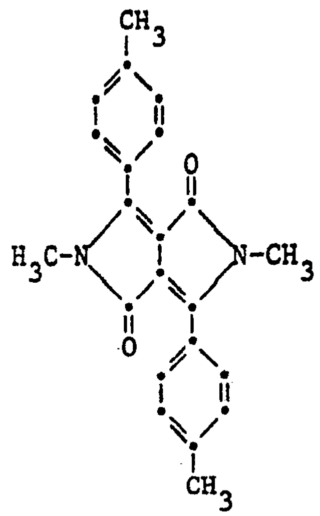

(XIV)

Beispiel 7: Eine Suspension von 5,8 Teilen 1,4-Diketo-3,6-diphenylpyrrolo-[3,4-c]-pyrrol in 60 Teilen Dimethylformamid wird unter Rühren bei 20°C mit 8,1 Teilen einer 30%igen methanolischen Lösung von Natriummethylat versetzt. Die Mischung wird 20 Minuten bei 25°C verrührt. Nach Abdestillieren von 15 Teilen Lösungsmittel bei 20 mbar, Abkühlen auf 20°C und Zugabe von 8,4 Teilen n-Butylbromid, wird die Mischung 20 Stunden bei 60°C, anschliessend 2 Stunden bei 100°C gerührt. Nach beendeter Reaktion wird das Gemisch in 500 Teile Wasser ausgetragen, die Mischung zum Sieden erhitzt und hierauf auf 10°C gekühlt. Das abfiltrierte Rohprodukt wird mit 100 Teilen Methanol zum Sieden erhitzt. Die Mischung wird heiss filtriert und der ungelöste Anteil wie das Filtrat gesondert aufgearbeitet.

Der ungelöste Anteil lässt sich aus einer reichlichen Menge Methanol kristallisieren. Das erhaltene Produkt ist 1,4-Diketo-2-n-butyl-3,6-diphenylpyrrolo-[3,4-c]-pyrrol vom Smp. 250-252°C (Formel XV). Dieser Farbstoff besitzt sehr gutes Ziehvermögen auf PES und liefert analog Beispiel 1b eine kräftige gelbe Färbung mit sehr guter Sublimier-und ausreichender Lichtechtheit.

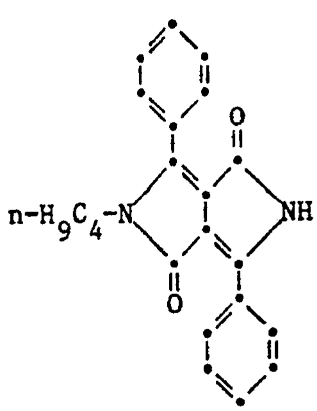

(XV)

Das aus dem abgekühlten Filtrat isolierte Produkt wird nochmals aus Cyclohexan kristallisiert. Das erhaltene 1,4-Diketo-2,5-di-n-butyl-3,6-diphenylpyrrolo-[3,4-c]-pyrrol vom Smp. 123-124°C (Formel XVI) ergibt nach der im Beispiel 1b angegebenen Färbemethode auf PES ein reines Gelb von sehr guter Sublimier- und guter Lichtechtheit.

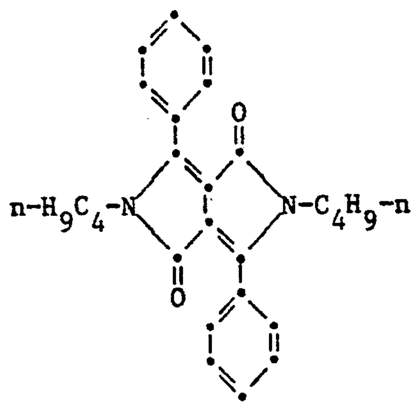

(XVI)

Beispiel 8: Eine Suspension von 5,8 Teilen 1,4-Diketo-3,6-diphenylpyrrolo-[3,4-c]-pyrrol in 70 Teilen Dimethylformamid wird bei 20° C mit 5,6 Teilen Kalium-t-butylat versetzt und 1 Stunde bei 20° C verrührt. Alsdann wird die Mischung mit 7,6 Teilen Benzylchlorid versetzt und 2 Stunden bei 65° C gehalten. Nach Abkühlen auf 20° C wird das Reaktionsprodukt abfiltriert, mit wenig Dimethylformamid, darauf mit Aceton und heissem Wasser gewaschen. Nach dem Trocknen erhält man 4,5 Teile 1,4-Diketo-2,5-dibenzyl-3,6-diphenylpyrrolo-[3,4-c]-pyrrol (Formel XVII), das, falls erforderlich, aus Dimethylformamid kristallisiert werden kann. Es bilden sich gelborange Kristalle vom Smp. 290-292° C.

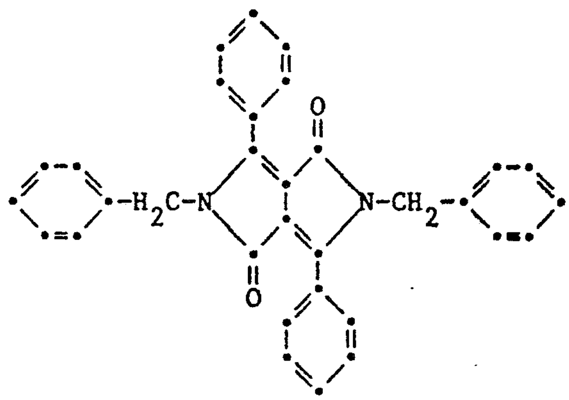

(XVII)

Beispiel 9: 5,8 Teile 1,4-Diketo-3,6-diphenylpyrrolo-[3,4-c]-pyrrol werden mit 50 Teilen Benzoylchlorid 22 Stunden bei 190° C verrührt. Das Ausgangsmaterial geht im Verlaufe der Reaktion in Lösung. Beim Abkühlen des Reaktionsgemisches fällt die Hauptmenge des Umsetzungsproduktes aus. Das Reaktionsgemisch wird unter Rühren in 320 Teilen Methanol eingetragen und die Mischung 2 Stunden ohne Beheizung gerührt. Das ausgefallene Produkt wird abfiltriert, mit Methanol gewaschen und getrocknet. Man erhält 4,9 Teile eines ockerfarbenen Rohproduktes, das durch Kristallisation aus 50 Teilen Dimethylformamid unter Zusatz eines Filtrierhilfsmittels gereinigt wird. Man erhält 3,8 Teile 1,4-Diketo-2,5-dibenzoyl-3,6-diphenylpyrrolo-[3,4-c]-pyrrol in Form bräunlichgelber Kristalle (Formel XVIII).

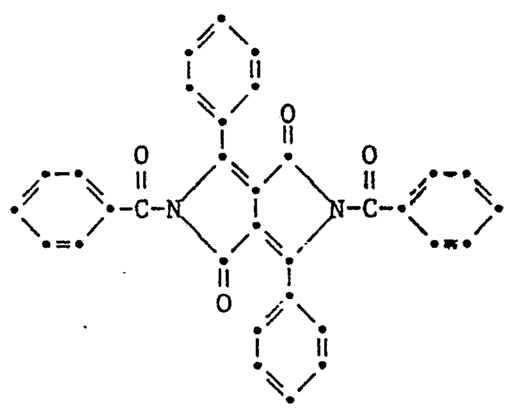

(XVIII)

Beispiel 10:

a) Die Mischung von 8,7 Teilen Bernsteinsäurediäthylester, 25 Teilen 4,4'-Dichlorbenzalanilin und 60 Teilen trockenem Dimethylformamid wird in einer Stickstoffatmosphäre bei -17 °C mit 0,5 Teilen Kalium-t-butylat versetzt. Die Temperatur steigt auf -11 °C an. Nach 15 Minuten Rühren wird bei -16 °C abermals 0,5 Teile Kalium-t-butylat zugegeben und anschliessend die Mischung 2 Stunden bei -10 bis -15 °C gerührt. Aus der ursprünglichen Suspension entsteht nach ca. 25 Minuten eine hellgelbe Lösung.

Nach Zugabe von 3 Teilen Eisessig, vermischt mit 15 Teilen Aethanol, wird die Mischung in 1000 Teile Wasser eingerührt. Nach halbstündigem Rühren wird der Niederschlag abfiltriert und unter Vakuum bei 70 °C getrocknet. Die erhaltenen 26 Teile eines Produktgemisches werden mit 420 Teilen Methanol 2 Stunden bei Siedetemperatur verrührt. Nach Erkalten der Suspension wird der Niederschlag abfiltriert, mit Methanol gewaschen und unter Vakuum bei 70 °C getrocknet.

Das vorgereinigte Produkt (16 Teile) wird mit 250 Teilen n-Butanol 10 Minuten bei Siedetemperatur verrührt. Man lässt die Suspension auf 80 °C abkühlen, filtriert darauf das ungelöste Produkt II (siehe unten) ab, kühlt das Filtrat auf 20 °C und filtriert das auskristallisierte Produkt I nach 1 Stunde ab. Nach Waschen mit Methanol und Trocknen erhält man 4,6 Teile des farblosen Produktes I mit dem Schmelzpunkt 257-258 °C. Durch Kristallisation aus n-Butanol erhält man das reine Produkt mit dem Schmelzpunkt 259-260 °C. Nach Analyse, Massen- ($M^+$ 580) und NMR-Spektrum entspricht es der Formel:

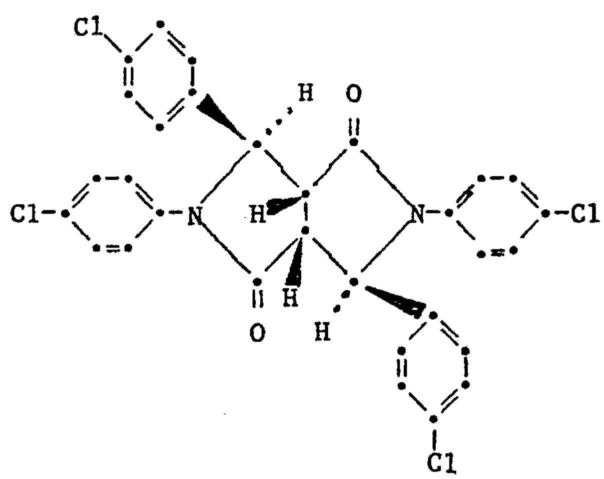

**(Produkt I)**

b) 1,45 Teile Produkt I, 1,7 Teile 2,3-Dichlor-5,6-dicyanbenzochinon (DDQ) und 40 Teile o-Dichlorbenzol werden 15 Stunden bei Siedetemperatur gerührt, darauf nach Zugabe von 1,7 Teilen DDQ weitere 15 Stunden am Sieden gehalten. Das Reaktionsgemisch wird darauf unter Zusatz von 40 Teilen Natriumhydroxidlösung 30 % und 15 Teilen Dithionit mittels Wasserdampfdestillation vom o-Dichlorbenzol befreit. Die zurückbleibende Suspension wird abfiltriert, der Niederschlag mit Wasser neutral gewaschen und getrocknet. Das Rohprodukt (1 Teil) wird in 70 Teilen Chlorbenzol bei Siedetemperatur gelöst und die Lösung nach Zugabe eines Filterhilfsmittels filtriert. Die Lösung wird unter vermindertem Druck auf etwa 1/4 des ursprünglichen Volumens eingeengt. Die nach 15-stündigem Stehenlassen abfiltrierten Kristalle werden nach Waschen mit Methanol mit 25 Teilen n-Butanol 10 Minuten bei Siedetemperatur verrührt. Das in n-Butanol schwer lösliche Produkt wird abfiltriert, mit n-Butanol sowie Methanol gewaschen und getrocknet. Man erhält 0,44 Teile 1,4-Diketo-2,5-di-(4'-chlorphenyl)-3,6-di-(4"-chlorphenyl)-pyrrolo-[3,4-c]-pyrrol in Form gelber, über 300 °C schmelzender Kristalle (Formel XIX).

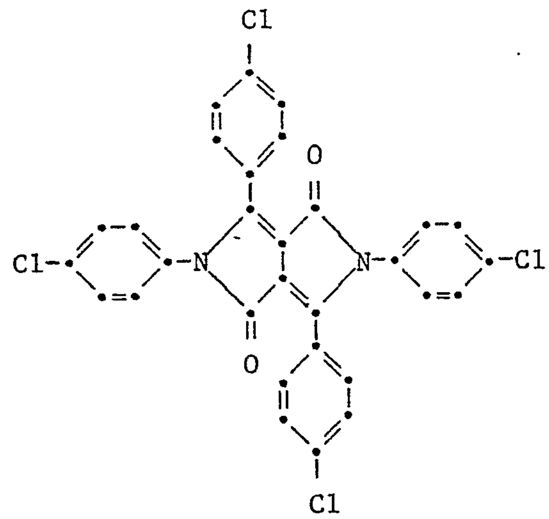

(XIX)

c) Produkt II zeigt nach Kristallisation aus Chlorbenzol einen Schmelzpunkt von 322-325° C. Es kommt ihm nach Analyse und Massenspektrum ($M^+$ 829) folgende Formel zu:

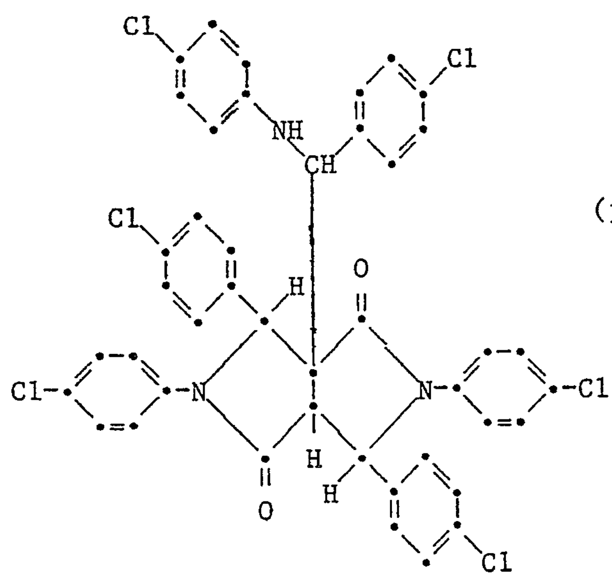

(Produkt II)

Beispiel 11: Eine auf 145° C erwärmte Mischung von 5,8 Teilen 1,4-Diketo-3,6-diphenyl-pyrrolo-[3,4-c]-pyrrol, 8,5 Teilen Natriumcarbonat und 60 Teilen Dimethylformamid wird innerhalb 30 Minuten mit einer Mischung von 9,8 Teilen Chloressigsäureäthylester und 20 Teilen Dimethylformamid versetzt. Die entstandene braungelbe Lösung wird 30 Minuten bei 140-145° C nachgerührt, abgekühlt und auf 1000 Teile Wasser von 15° C ausgetragen. Nach einstündigem Stehenlassen bei 20° C wird die wässrige Phase abgegossen und das Rohprodukt mit 200 Teilen Aethanol zum Sieden erhitzt. Die entstandene Suspension wird bei 20° C filtriert, das Produkt mit Aethanol, darauf mit Wasser von 90° C gewaschen und getrocknet. Man erhält 5,6 Teile eines gelben Produktes mit dem Schmelzpunkt 204-208° C. Durch Kristallisation aus Toluol erhält man reines 1,4-Diketo-2,5-di-(carbäthoxymethyl)-3,6-diphenyl-pyrrolo-[3,4-c]-pyrrol der nachstehenden Formel mit dem Schmelzpunkt 209-210° C und $M^+$ 460.

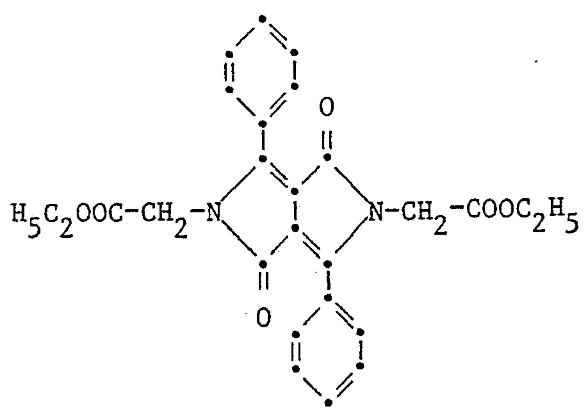

Beispiel 12: 5,8 Teile 1,4-Diketo-3,6-diphenyl-pyrrolo-[3,4-c]-pyrrol werden in 270 Teilen wasserfreiem Dimethylformamid suspendiert und unter Rühren mit 8 Teilen Natriummethylatlösung 30 % versetzt. Die Mischung wird 2 Stunden bei 70-75 °C verrührt und darauf die violette Lösung auf 20 °C gekühlt. Unter einem Druck von 20 mbar werden 30 Teile Dimethylformamid bei ca. 45 °C abdestilliert. Nach Belüftung werden 4,6 Teile Chloracetonitril zugefügt und die Mischung 10 Stunden bei 85 - 90 °C verrührt. Die braungelbe, etwas trübe Lösung wird darauf unter vermindertem Druck auf 1/5 des ursprünglichen Volumens eingeengt. Die zurückbleibende Lösung wird in 500 Teile Wasser eingerührt und die Suspension 1 Stunde gerührt. Der Niederschlag wird abfiltriert, mit Wasser gewaschen und getrocknet. Zur Vorreinigung wird das gepulverte Produkt mit 80 Teilen Methanol 1 Stunde bei Siedetemperatur verrührt. Das abfiltrierte, mit Methanol gewaschene und getrocknete Produkt wird mit 100 Teilen Eisessig 1 Stunde bei Siedetemperatur verrührt. Die Suspension wird heiss filtriert; Filtrat und Produkt werden separat aufgearbeitet.

Das Eisessigfiltrat wird auf ein kleines Volumen eingeengt und abgekühlt. Der ausgefallene Niederschlag wird abfiltriert und zweimal aus wenig Dimethylformamid umkristallisiert. Das gereinigte, bis 300 °C nicht schmelzende gelbe Produkt entspricht nach Analyse der Formel:

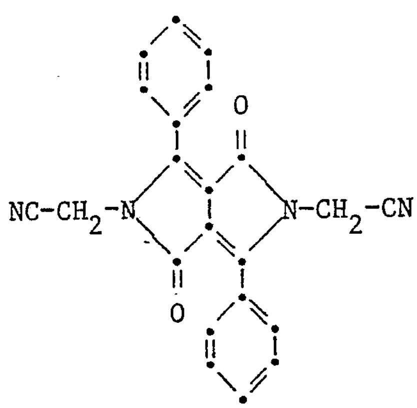

[1,4-Diketo-2,5-di-(cyanmethyl)-3,6-diphenyl-pyrrolo-[3,4-c]-pyrrol].

Der in Eisessig nicht lösliche Anteil wird mit 70 Teilen Dimethylformamid 1 Stunde bei Siedetemperatur gerührt, das ungelöste, nicht umgesetzte Ausgangsmaterial (2 Teile) bei 100 °C abfiltriert. Das Filtrat wird unter vermindertem Druck zur Trockene eingedampft. Der Rückstand wird in 520 Teilen o-Dichlorbenzol bei Siedetemperatur gelöst. Ein geringer ungelöster Anteil wird abfiltriert und die Lösung erkalten gelassen. Man erhält 0,35 Teile eines Produktes, dem nach Analyse die folgende Formel

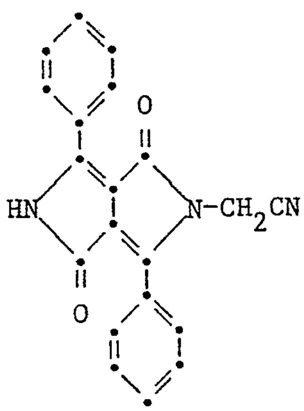

[1,4-Diketo-2-cyanmethyl-3,6-diphenyl-pyrrolo-[3,4-c]-pyrrol]
zukommt. Der Farbstoff färbt PES nach der in Beispiel 1b angegebenen Methode in gelben Tönen; sein Schmelzpunkt liegt über 310 °C.

Beispiel 13: Zur Suspension von 5,8 Teilen 1,4-Diketo-3,6-diphenyl-pyrrolo-[3,4-c]-pyrrol und 8,5 Teilen Natriumcarbonat in 100 Teilen Dimethylformamid werden bei 140-142 °C unter Rühren 4,0 Teile α,α-Dichlor-m-xylol, gelöst in 20 Teilen Dimethylformamid, in 40 Minuten zugetropft. Nach vierstündigem Rühren bei 140 - 142 °C wird die Mischung abgekühlt. Das abfiltrierte Produkt und das Filtrat werden separat aufgearbeitet.

Das abfiltrierte Produkt wird mit heissem Wasser gewaschen und getrocknet. Nach Kristallisation aus Dimethylformamid wird eine geringe Menge eines gelben Produktes erhalten, dem nach Analyse die Struktur:

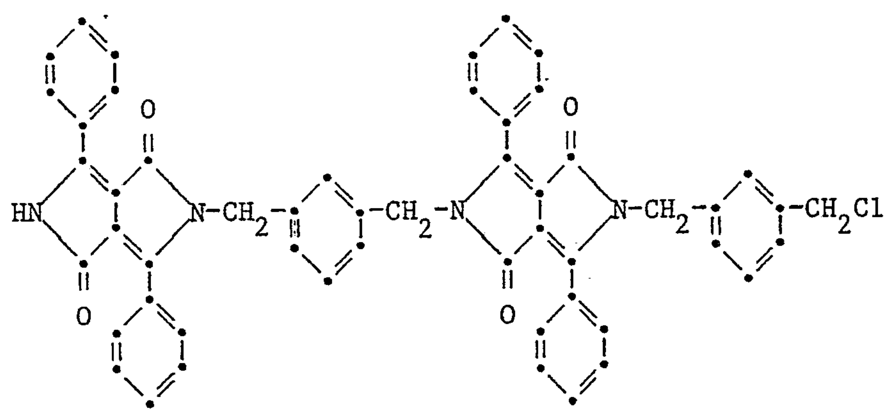

zukommt. Elementaranalyse:

| | | | | |
|---|---|---|---|---|
| Ber. für $C_{52}H_{37}ClN_4O_4$ (in %): | C 76,41 | H 4,56 | N 6,86 | % |
| Gef. (in %) | C 75,4 | H 4,7 | N 7,0 | % |

Das oben erhaltene Filtrat wird nach 20-stündigem Stehenlassen von einer geringen Menge ausgefallenem Ausgangsmaterial abfiltriert und das Filtrat unter Vakuum zur Trockene eingedampft. Der Rückstand wird in Methanol aufgenommen, abfiltriert und das erhaltene gelbe Produkt mit Methanol gewaschen. Es wird nach Zerkleinerung mit Wasser und Methanol gewaschen, schliesslich im Vakuum bei 70 °C getrocknet. Das erhaltene Produkt stellt ein Gemisch dar. Es sintert bei 190 °C und schmilzt unter allmählicher Zersetzung ab 210 °C.

Durch Kristallisation aus wenig Dimethylformamid lässt sich ein Produkt isolieren, dem nach Analyse die Formel zukommt:

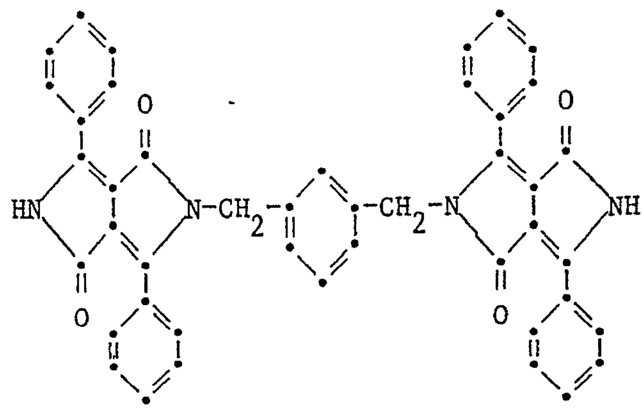

Elementaranalyse:

| | | | | |
|---|---|---|---|---|
| Ber. für $C_{44}H_{30}N_4O_4$ (in %): | C 77,86 | H 4,46 | N 8,26 | % |
| Gef. (in %) : | C 77,1 | H 4,8 | N 7,8 | %. |

Beispiele 14: Die auf 140-142 °C erwärmte Mischung von 5,8 Teilen 1,4-Diketo-3,6-diphenyl-pyrrolo-[3,4-c]-pyrrol, 12,2 Teilen Kaliumcarbonat und 60 Teilen Dimethylformamid wird im Laufe von 30 Minuten unter Rühren mit einer Lösung von 16 Teilen Toluol-4-sulfonsäureäthylester in 15 Teilen Dimethylformamid versetzt. Die entstandene braungelbe Lösung wird 5 Minuten bei 142 °C nachgerührt und bei 70 °C mit 100 Teilen Methanol versetzt. Der ausgefallene gelbe Niederschlag wird bei 15 °C abfiltriert, mit Methanol sowie mit 90 °C heissem Wasser gewaschen und getrocknet. Das Rohprodukt (3,6 Teile) wird zuerst aus 40 Teilen Dimethylformamid, darauf aus 100 Teilen n-Butanol umkristallisiert. Es werden 2,8 Teile 1,4-Diketo-2,5-diäthyl-3,6-diphenyl-pyrrolo-[3,4-c]-pyrrol der nachstehenden Formel in Form orangegelber Nadeln vom Schmelzpunkt 229-230 °C erhalten:

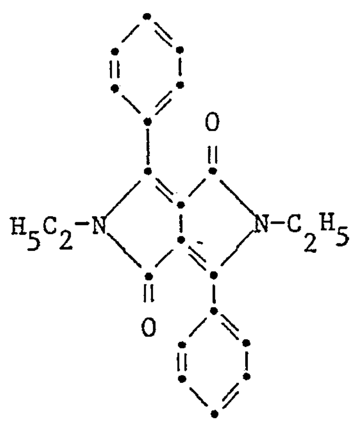

Beispiel 15: Die Suspension von 7,2 Teilen 1,4-Diketo-3,6-di-(4'-chlorphenyl)-pyrrolo-[3,4-c]-pyrrol und 11,2 Teilen Kaliumcarbonat in 80 Teilen Dimethylformamid wird unter Rühren bei 143 °C im Laufe von 25 Minuten mit einer Lösung von 11,2 Teilen Toluol-4-sulfonsäuremethylester in 20 Teilen Dimethylformamid versetzt. Nach weiteren 30 Minuten Rühren bei 142-145 °C wird nochmals eine Losung von 6 Teilen Toluol-4-sulfonsäuremethylester in 10 Teilen Dimethylformamid im Laufe von 10 Minuten zugefügt. Man hält die erhaltene Reaktionsmischung weitere 20 Minuten bei 142-145 °C und kühlt schliesslich auf 20 °C ab. Der Niederschlag wird abfiltriert, zweimal mit etwas Dimethylformamid, darauf mit Aethanol und 90 °C heissem Wasser nachgewaschen und getrocknet. Das Rohprodukt (3,4 Teile) wird in 550 Teilen Chlorbenzol bei Siedetemperatur gelöst. Aus der klärfiltrierten Lösung kristallisiert das gereinigte Produkt aus. Nach mehrstündigem Stehenlassen bei 20 °C wird das 1,4-Diketo-2,5-dimethyl-3,6-di-(4'-chlorphenyl)-pyrrolo-[3,4-c]-pyrrol abgetrennt. Man erhält 3 Teile orange Kristalle vom Fp. 337-339 °C der nachstehenden Formel

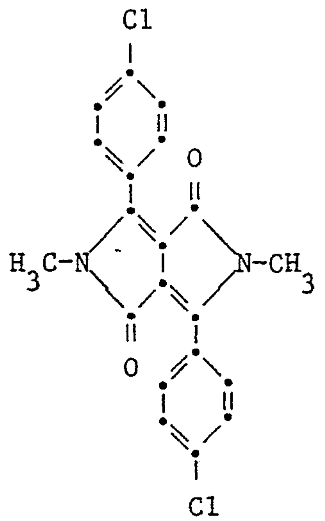

Beispiel 16: 7,2 Teile 1,4-Diketo-3,6-di-(4'-chlorphenyl)-pyrrolo-[3,4-c]-pyrrol und 11,2 Teile Kaliumcarbonat werden in 80 Teilen Dimethylformamid unter Rühren suspendiert. Die auf 141 °C erwärmte Mischung wird im Laufe von 45 Minuten mit einer Lösung von 16 Teilen Toluol-4-sulfonsäureäthylester in 20 Teilen Dimethylformamid versetzt. Man rührt das Gemisch 30 Minuten bei 145 °C nach und kühlt schliesslich auf 20 °C. Der abfiltrierte Niederschlag wird zuerst mit etwas Dimethylformamid, darauf mit Methanol sowie 90 °C heissem Wasser gewaschen und getrocknet. Das gepulverte Rohprodukt wird mit 200 Teilen n-Butanol 2 Stunden bei Siedetemperatur verrührt. Der Ausgangsmaterial enthaltende Niederschlag wird abfiltriert. Aus dem Filtrat scheidet sich beim Abkühlen 1 Teil orangegelbe Kristalle aus. Das 1,4-Diketo-2,5-diäthyl-3,6-di-(4'-chlorphenyl)-pyrrolo-[3,4-c]-pyrrol der nachstehenden Formel schmilzt bei 276 - 277 °C.

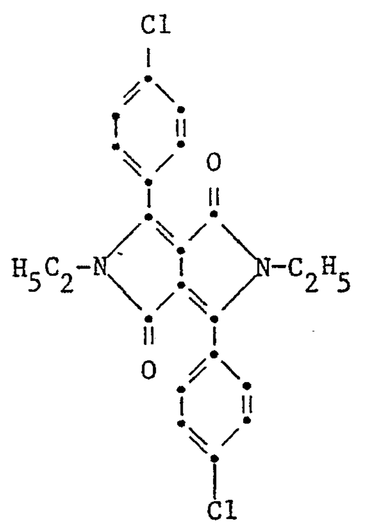

Beispiele 17: 7,2 Teile 1,4-Diketo-3,6-di-(4'-chlorphenyl)-pyrrolo-[3,4-c]-pyrrol und 11,2 Teile Kaliumcarbonat werden unter Rühren in 80 Teilen Dimethylformamid suspendiert. Die auf 142 °C erwärmte Mischung wird im Laufe von 50 Minuten mit einer Mischung von 11 Teilen 1-Brombutan und 10 Teilen Dimethylformamid versetzt und anschliessend 30 Minuten bei 142 °C nachgerührt. Nach Abkühlen auf 25 °C wird die Reaktionsmischung filtriert, der Niederschlag mit 20 Teilen Dimethylformamid gewaschen und das Filtrat unter vermindertem Druck eingedampft. Der noch warme Rückstand wird mit Methanol versetzt, die entstandene Suspension auf 20 °C gekühlt und filtriert. Das erhaltene Produkt wird mit Methanol, darauf mit 90 °C heissem Wasser gewaschen und getrocknet. Man erhält 3,9 Teile eines Rohproduktes, das nach Kristallisation aus 80 Teilen n-Butanol 3,3 Teile 1,4-Diketo-2,5-di-n-butyl-3,6-di-(4'-chlorphenyl)-pyrrolo-[3,4-c]-pyrrol der nachstehenden Formel in Form orangeroter Kristalle vom Fp. 176-177 °C liefert.

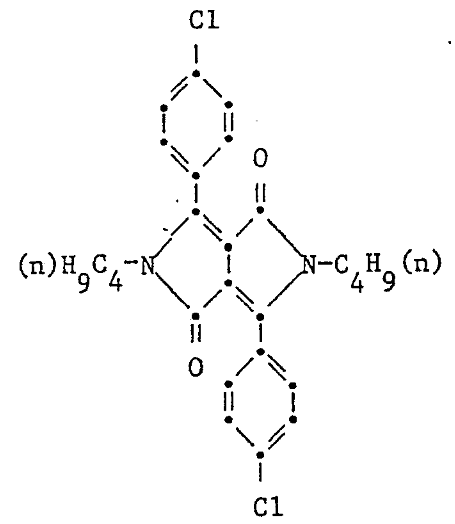

## Ansprüche

**1.** Verbindungen der Formel I

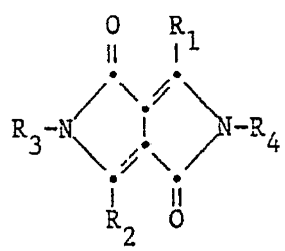

(I),

worin $R_1$ und $R_2$ einen Rest der Formel II

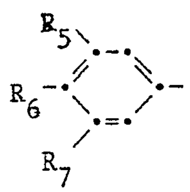

(II)

bedeuten, worin $R_5$, $R_6$ und $R_7$ unabhängig voneinander Wasserstoff, Halogen, Carbamoyl, Cyano, Trifluormethyl, $c_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxycarbonyl, $C_2$-$C_{13}$-Alkanoylamino, $C_1$-$C_{12}$,-Monoalkylamino, $C_2$-$C_{24}$-Dialkylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_5$, $R_6$ und $R_7$ Wasserstoff bedeutet, und $R_3$ und $R_4$ unabhängig voneinander $C_1$-$C_{12}$-Alkyl, Cyanmethyl, $C_2$-$C_{13}$-Alkoxycarbonyl, Carbamoyl, $C_2$-$C_{13}$-Alkyl-carbamoyl, $C_2$-$C_{13}$-Alkyl-$C_1$-$C_4$-alkoxycarbonyl, unsubstituiertes oder durch Halogen, $C_1$-$C_{12}$-Alkyl, $C_1$-$C_{12}$-Alkoxy, Trifluormethyl oder Nitro substituiertes Phenyl, Benzoyl oder Benzyl, oder die Gruppe der Formel

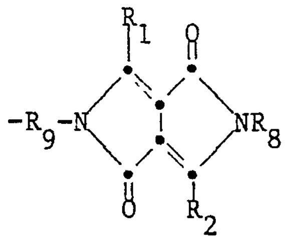

bedeuten, worin $R_1$ und $R_2$ die oben angegebene Bedeutung haben, $R_8$ Wasserstoff bedeutet und $R_9$ einen zweiwertigen Rest bedeutet, der sich von einem $C_1$-$C_8$-Dihaloalkan, $C_1$-$C_{10}$-Dicarbonsäurehalogenid, Phenylendihalogenid oder Xylylendihalogenid ableitet, wobei entweder $R_3$ oder $R_4$ auch Wasserstoff sein kann.

**2.** Verbindungen der Formel I gemäss Anspruch 1, worin $R_3$ und $R_4$ beide verschieden von Wasserstoff sind und den gleichen Substituenten bedeuten.

**3.** Verbindungen der Formel I gemäss Anspruch 1, worin $R_1$ und $R_2$ einen Rest der Formel III

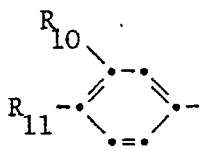

(III)

bedeuten, worin einer der Substituenten $R_{10}$ und $R_{11}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes Phenylcarbamoyl bedeutet, und der andere Wasserstoff ist.

**4.** Verbindungen der Formel I gemäss Anspruch 1, worin $R_3$ und $R_4$ $C_1$-$C_8$-Alkyl, $C_2$-$C_5$-Alkoxycarbonyl, Carbamoyl, $C_2C_{13}$-Alkyl-$C_1$-$C_4$-alkoxycarbonyl, unsubstituiertes oder durch Chlor, Brom, $C_1$-$C_4$-Alkyl, $C_1$-$C_4$-Alkoxy, Trifluormethyl oder Nitro substituiertes Phenyl bedeuten.

**5.** Verbindungen der Formel I gemäss Anspruch 1, worin $R_1$ und $R_2$ einen Rest der Formel IV

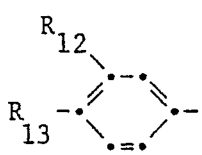

(IV)

bedeuten, worin einer der Substituenten $R_{12}$ und $R_{13}$ $C_1$-$C_4$-Alkyl und der andere Wasserstoff bedeutet, und $R_3$ und $R_4$ $C_1$-$C_8$-Alkyl bedeuten.

**6.** Verbindungen der Formel I gemäss Anspruch 1, worin $R_1$ und $R_2$ einen Rest der Formel V

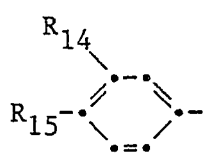

(V)

bedeuten, worin einer der Substituenten $R_{14}$ und $R_{15}$ Cyano und der andere Wasserstoff bedeuten, und $R_3$ und $R_4$ $C_1$-$C_4$-Alkyl bedeuten.

**7.** Verwendung der Verbindungen der Formel I gemäss Anspruch 1 als polymerlösliche Farbstoffe oder

Pigmente für hochmolekulares organisches Material.

8. Verwendung der Verbindungen der Formel I gemäss Anspruch 1 als polymerlösliche Farbstoffe für Polyester.

9. Hochmolekulares organisches Material enthaltend eine Verbindung der Formel I gemäss Anspruch 1.

**Claims**

1. A compound of the formula I

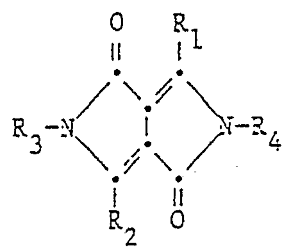

(I),

in which $R_1$ and $R_2$ are radicals of the formula II

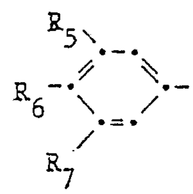

(II)

in which $R_5$, $R_6$ and $R_7$ independently of one another are hydrogen, halogen, carbamyl, cyano, trifluoromethyl, $C_2$-$C_{13}$alkylcarbamyl, $C_1$-$C_{12}$alkyl, $C_1$-$C_{12}$alkoxy, $C_{1-12}$alkylmercapto, $C_2$-$C_{13}$alkoxycarbonyl, $C_2$-$C_{13}$alkanoylamino, $C_1$-$C_{12}$monoalkylamino, $C_2$-$C_{24}$dialkylamino, or phenoxy, phenylmercapto, phenoxycarbonyl, phenylcarbamyl or benzoylamino which are unsubstituted or substituted by halogen, $C_1$-$C_{12}$alkyl or $C_1$-$C_{12}$alkoxy, at least one of the substituents $R_5$, $R_6$ and $R_7$ being hydrogen, in which $R_3$ and $R_4$ independently of one another are $C_1$-$C_{12}$alkyl, cyanomethyl, $C_2$-$C_{13}$alkoxycarbonyl, carbamyl, $C_2$-$C_{13}$alkylcarbamyl $C_2$-$C_{13}$alkyl-$C_1$-$C_4$alkoxycarbonyl, or phenyl, benzoyl or benzyl which is unsubstituted or substituted by halogen, $C_1$-$C_{12}$alkyl, $C_1$-$C_{12}$alkoxy, trifluoromethyl or nitro, or the group of the formula

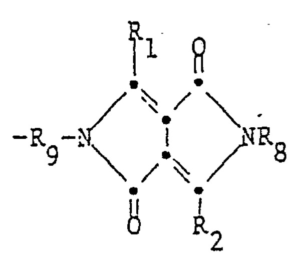

in which $R_1$ and $R_2$ are as defined above, $R_8$ is hydrogen and $R_9$ is a divalent radical derived from a $C_1$-$C_8$dihaloalkane, $C_1$-$C_{10}$dicarboxylic acid halide, phenylene dihalide or xylylene dihalide, and in which either $R_3$ or $R_4$ can also be hydrogen.

2. A compound of the formula I according to claim 1, in which both $R_3$ and $R_4$ are different from hydrogen and are the same substituents.

3. A compound of the formula I according to claim 1, in which $R_1$ and $R_2$ are radicals of the formula III

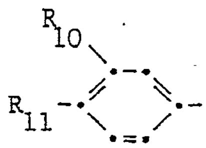

(III)

in which one of the substituents $R_{10}$ and $R_{11}$ is chlorine, bromine, $C_1$-$C_4$alkyl, cyano, $C_1$-$C_4$alkoxy, phenoxy which is unsubstituted or substituted by chlorine or methyl, carbamyl, $C_2$-$C_5$alkylcarbamyl or phenylcarbamyl which is unsubstituted or substituted by chlorine, methyl or methoxy, and the other substituent is hydrogen.

4. A compound of the formula I according to claim 1, in which $R_3$ and $R_4$ are $C_1$-$C_8$alkyl, $C_2$-$C_5$alkoxycarbonyl, carbamyl, $C_2$-$C_{13}$alkyl-$C_1$-$C_4$alkoxycarbonyl, or phenyl which is unsubstituted or substituted by chlorine, bromine, $C_1$-$C_4$alkyl, $C_1$-$C_4$alkoxy, trifluoromethyl or nitro.

5. A compound of the formula I according to claim 1, in which $R_1$ and $R_2$ are radicals of the formula IV.

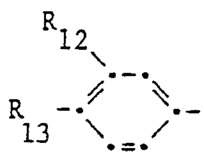

(IV)

in which one of the substituents $R_{12}$ and $R_{13}$ is $C_1$-$C_4$alkyl and the other is hydrogen, and $R_3$ and $R_4$ are $C_1$-$C_8$alkyl.

6. A compound of the formula I according to claim 1, in which $R_1$ and $R_2$are radicals of the formula

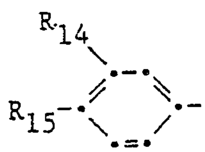

(V)

in which one of the substituents $R_{14}$ and $R_{15}$ is cyano and the other is hydrogen, and $R_3$ and $R_4$ are $C_1$-$C_4$alkyl.

7. The use of a compound of the formula I according to claim 1 as a polymer-soluble dye or pigment for a high molecular weight organic material.

8. The use of a compound of the formula I according to claim 1 as a polymer-soluble dye for polyester.

9. A high molecular weight organic material containing a compound of the formula I according to claim 1.

**Revendications**

1. Composés répondant à la formule I :

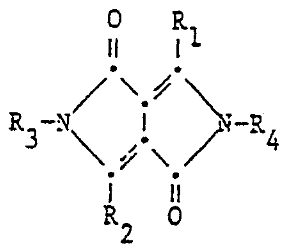

(I),

dans laquelle

$R_1$ et $R_2$ représentent chacun un radical de formule II :

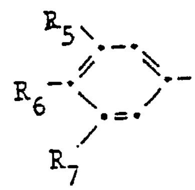

(II)

dans lequel $R_5$, $R_6$ et $R_7$ représentent chacun, indépendamment l'un de l'autre, l'hydrogène, un halogène, un carbamoyle, un cyano, un trifluorométhyle, un alkylcarbamoyle en $C_2$-$C_{13}$, un alkyle en $C_1$-$C_{12}$, un alcoxy en $C_1$-$C_{12}$, un alkylthio en $C_1$-$C_{12}$, un alcoxycarbonyle en $C_2$-$C_{13}$, un alcanoylamino en $C_2$-$C_{13}$, un monoalkylamino en $C_1$-$C_{12}$, un dialkylamino en $C_2$-$C_{24}$ ou un radical phénoxy, phénylthio, phénylcarbonyle, phénylcarbamoyle ou benzoylamino non substitué ou porteur d'un halogène, d'un alkyle en $C_1$-$C_{12}$ ou d'un alcoxy en $C_1$-$C_{12}$, au moins un des symboles $R_5$, $R_6$ et $R_7$ représentant l'hydrogène, et

$R_3$ et $R_4$ représentent chacun, indépendamment l'un de l'autre, un alkyle en $C_1$-$C_{12}$, un cyanométhyle, un alcoxycarbonyle en $C_2$-$C_{13}$, un carbamoyle, un alkylcarbamoyle en $C_2$-$C_{13}$, un ($C_2$-$C_{13}$-alkyl)-($C_1$-$C_4$-alcoxy)-carbonyle, un radical phényle, benzoyle ou benzyle non substitué ou porteur d'un halogène, d'un alkyle en $C_1$-$C_{12}$, d'un alcoxy en $C_1$-$C_{12}$, d'un trifluorométhyle ou d'un nitro, ou un radical de formule :

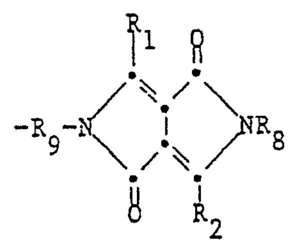

dans lequel $R_1$ et $R_2$ ont les significations qui leur ont été données ci-dessus, $R_8$ représente l'hydrogène et $R_9$ représente un radical bivalent dérivant d'un dihalogénoalcane en $C_1$-$C_8$, d'un halogénure d'acide dicarboxylique en $C_1$-$C_{10}$, d'un dihalogénure de phénylène ou d'un dihalogénure de xylylène,

et l'un des symboles $R_3$ et $R_4$ peut également représenter l'hydrogène.

**2.** Composés de formule I selon la revendication 1, dans lesquels $R_3$ et $R_4$ ne sont ni l'un ni l'autre l'hydrogène et représentent les mêmes substituants.

**3.** Composés de formule I selon la revendication 1, dans lesquels $R_1$ et $R_2$ représentent chacun un radical répondant à la formule III :

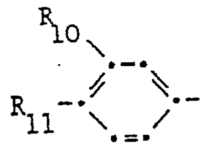

(III)

dans laquelle l'un des substituants $R_{10}$ et $R_{11}$ représente le chlore, le brome, un alkyle en $C_1$-$C_4$, un cyano, un alcoxy en $C_1$-$C_4$, un phénoxy non substitué ou porteur d'un chlore ou d'un méthyle, un carbamoyle, un alkylcarbamoyle en $C_2$-$C_5$, ou un phénylcarbamoyle non substitué ou porteur d'un chlore, d'un méthyle ou d'un méthoxy, et l'autre représente l'hydrogène.

**4.** Composés de formule I selon la revendication 1, dans lesquels $R_3$ et $R_4$ représentent chacun un alkyle en $C_1$-$C_8$, un alcoxycarbonyle en $C_2$-$C_5$, un carbamoyle, un ($C_2$-$C_{13}$-alkyl)-($C_1$-$C_4$-alcoxy)-carbonyle, ou un phényle non substitué ou porteur d'un chlore, d'un brome, d'un alkyle en $C_1$-$C_4$, d'un alcoxy en $C_1$-$C_4$, d'un trifluorométhyle ou d'un nitro.

**5.** Composés de formule I selon la revendication 1, dans lesquels $R_1$ et $R_2$ représentent chacun un radical répondant à la formule IV :

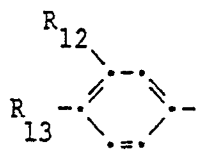

(IV)

dans laquelle l'un des substituants $R_{12}$ et $R_{13}$ représente un alkyle en $C_1$-$C_4$ et l'autre l'hydrogène, et $R_3$ et $R_4$ représentent chacun un alkyle en $C_1$-$C_8$.

**6.** Composés de formule I selon la revendication 1, dans lesquels $R_1$ et $R_2$ représentent chacun un radical répondant à la formule V :

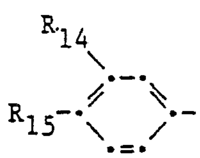

(V)

dans laquelle l'un des symboles $R_{14}$ et $R_{15}$ représente un cyano et l'autre l'hydrogène, tandis que $R_3$ et $R_4$ représentent chacun un alkyle en $C_1$-$C_4$.

**7.** Application des composés de formule I selon la revendication 1 comme colorants solubles dans les polymères ou comme pigments, pour des matières organiques à haut poids moléculaire.

**8.** Application des composés de formule I selon la revendication 1 comme colorants solubles dans les polymères pour des polyesters.

**9.** Matières organiques à haut poids moléculaire qui contiennent un composé de formule I selon la revendication 1.